# EUROPÄISCHE PATENTSCHRIFT

(11) **EP 1 332 218 B1**
(45) Veröffentlichungstag und Bekanntmachung des Hinweises auf die Patenterteilung: **14.03.2012**
(21) Anmeldenummer: 01993692.1
(22) Anmeldetag: 31.10.2001
(51) Int. Cl.: C12N 15/62, C12Q 1/68, C12Q 1/70

(54) **DIFFERENZIERUNG AUSLÖSENDE SUBSTANZEN**
DIFFERENTIATION-INDUCING SUBSTANCES
SUBSTANCES DECLENCHANT UNE DIFFERENCIATION

(30) Priorität: 11.11.2000 DE 10056059
(43) Veröffentlichungstag der Anmeldung: 06.08.2003
(73) Patentinhaber: Heart BioSystems GmbH, 69120 Heidelberg (DE)
(72) Erfinder: KÜPPER, Jan-Heiner, 47574 Goch (DE); KANDOLF, Reinhard, 72379 Hechingen (DE); MEYER, Ralph, 55596 Waldböckelheim (DE)
(74) Vertreter: Simandi, Claus
(86) Internationale Anmeldenummer: PCT/EP2001/012660
(87) Internationale Veröffentlichungsnummer: WO 2002/038778

(56) Entgegenhaltungen:
- DATABASE [Online] pHygEGFP, Clontech Catalog #6014-1, 30. August 2000 (2000-08-30) retrieved from HTTP://WWW.CLONTECH.COM/TECHINFO/VECTORS/V ECTORSF-I/PHYGEGFP.SHTML#USE XP002193931
- DATABASE [Online] April 1999 (1999-04) LEDO: "Development of a Transgenic Model to Assess Bioavailable Genotixicity in Sediments" retrieved from HTTP://WWW.WES.ARMY.MIL/EL/DOTS/PDFS/EEDP0 1-43.PDF XP002193932
- PRIMIG M ET AL: "A novel GFPneo vector designed for the isolation and analysis of enhancer elements in transfected mammalian cells" GENE, ELSEVIER BIOMEDICAL PRESS. AMSTERDAM, NL, Bd. 215, Nr. 1, Juli 1998 (1998-07), Seiten 181-189, XP004149242 ISSN: 0378-1119
- LIU LI ET AL: "In vivo gene repair of point and frameshift mutations directed by chimeric RNA/DNA oligonucleotides and modified single-stranded oligonucleotides." NUCLEIC ACIDS RESEARCH, Bd. 29, Nr. 20, 15. Oktober 2001 (2001-10-15), Seiten 4238-4250, XP002193930 ISSN: 0305-1048

## Beschreibung

Die vorliegende Erfindung betrifft ein Verfahren zur Identifizierung von Substanzen, die bei eukaryontischen Zellen eine Differenzierung auslösen, in dem Verfahren verwendete DNA-Konstrukte, Plasmide, Viren und Zellinien, sowie die Verwendung der identifizierten Substanzen.

Die Differenzierung von Zellen aus Stammzellen ist ein allgemeines biologisches Phänomen während der Embryonalentwicklung, spielt aber auch im adulten Organismus eine sehr große Rolle bei Regenerationsprozessen (z.B. Hautregeneration, Blutbildung, Regeneration der Darmepithelien, Leberregeneration nach Vergiftung oder Alkoholabusus etc.). Wie bei allen wichtigen biologischen Prozessen können Störungen chronische Erkrankungen auslösen oder lethal sein.

Bei der Tumorentstehung kommt es sehr oft, wenn nicht gar immer, zu einer "Rück"-Differenzierung von Zellen, d.h. Zellen gehen in einen undifferenzierten, embryonalen Zustand zurück. Trotz großer methodischer Verbesserungen bei Früherkennung und Therapie von Tumorerkrankungen, ist die Mortalität immer noch sehr hoch und der Leidensdruck bei betroffenen Patienten immens. Daher sind auch Entdeckung und Entwicklung neuer und wirksamerer Krebsmedikamente (Zytostatika) von herausragender gesundheitspolitischer Bedeutung und haben - aus der Sicht der pharmazeutischen Industrie - ein sehr großes Wächstumspotential auf dem Arzneimittelmarkt.

Da sich auf dem Weg von einer normalen gesunden Zellen bis zu einer Tumorzelle viele genetische Veränderungen ereignen, gibt es prinzipiell auch viele Möglichkeiten, in den Stoffwechsel von Tumorzellen einzugreifen und damit Wachstum und Ausbreitung zu verhindern. So bewirkt die größte Klasse der Zytostatika eine Schädigung der DNA von Zellen. Dies führt nach der Zellteilung zu tödlichen Mutationen, oder die Zelle stirbt direkt durch Auslösung des sogenannten programmierten Zelltods (Apoptose) ab. Davon sind alle schnell wachsenden Zellen betroffen, also nicht nur Tumorzellen sondern auch gesunde wachsende Zellen.

Eine weitere Klasse von Zytostatika ist die der Antimetabolite, die den Stoffwechsel schnell wachsender Zellen lahmlegen. Eine dritte Klasse von Zytostatika schädigt den sogenannten Spindelfaserapparat von teilenden Zellen. Dadurch wird die Zellteilung gehemmt bzw. die Zellen werden direkt abgetötet. Eine vierte und relativ neue Klasse ist die der anti-angiogenetischen Substanzen, diese interferieren mit der Fähigkeit von Tumorzellen, neue Blutgefäße zur eigenen Versorgung zu induzieren.

Eine im Zusammenhang mit der vorliegenden Erfindung relevante, relativ neue Klasse von Zytostatika greift in das Expressionsmuster von Genen ein, die z.T. während der Tumorentstehung abgeschaltet wurden. Dies sind häufig sogenannte Tumorsuppressorgene und Differenzierungsgene. Die erneute Induktion und Expression dieser Gene führt meistens zu einem Verlust der Wachstumsvorteile einer Krebszelle und kann diese auch leichter angreifbar machen für das Immunsystem.

Es ist seit langem bekannt, daß solche Differenzierung auslösenden Substanzen mit dem Methylierungsstatus sogenannter "CpG-Inseln" interferieren. CpG-Inseln finden sich besonders häufig in den 5'-regulatorischen Sequenzen von Genen und spielen eine wichtigen Rolle beim genomischen Imprinting, d.h. bei der Regulation paternaler versus maternaler Genexpression. Durch Methylierung der in Promotoren vorhandenen CpG-Inseln können Gene abgeschaltet werden. Wenn die für die Methylierung hauptverantwortliche DNA-Methylase inaktiv ist, kommt es zu lethalen Enwicklungsstörungen, wie in einem Knockout-Modell der Maus gezeigt werden kann.

Man geht daher heute davon aus, daß eine genetisch gesteuerte Balance von Methylierung und Demethylierung in der Embryogenese besteht. Eine Störung dieser Balance liegt oft in Tumorzellen vor. So wurde nachgewiesen, daß Promotoren von Tumorsuppressorgenen durch Methylierung der CpG-Inseln abgeschaltet werden können, weshalb z.B. die Hypomethylierung induzierende Substanz 5'-Azacytidin (Aza) als potentes Zytostatikum bei vielen Tumorarten wirkt.

Eine weiterer für die Differenzierung wichtiger Mechanismus ist die Beeinflussung der Histonacetylierung. Histone sind DNAbindende Proteine, welche die Chromatinstruktur regulieren und auch Genexpression beeinflussen können. Dies geschieht vorzugsweise über biochemische Modifikationen dieser Histone, z.B. durch Acetylierungen oder Phosphorilierungen. So ist bekannt, daß die Substanz Trichostatin A (TSA) ein spezifischer Inhibitor der Histondeacetylase ist. Es ist vor kurzem gezeigt worden, daß Deacetylierung des Histons H4 zu Chromatinkondensation führt und dadurch Genexpression unterdrückt werden kann. Dementsprechend führt die Inhibition der Histondeacetylase durch TSA zu Chromatindekondensation und kann dadurch die Unterdrückung der Genexpression aufheben.

Man muß davon ausgehen, daß es noch viele weitere, bisher unbekannte Mechanismen gibt, mit denen Tumorzellen die Expression der für sie gefährlichen Differenzierungs- und Tumorsuppressorgene abschalten können.

Während es für einige Klassen von Zytostatika bereits sehr gute Testsysteme gibt, mit denen sich auch neue Verbindungen identifizieren lassen, fehlen derartige Verfahren jedoch für die eingangs erwähnte Identifizierung von Substanzen, die eine Differenzierung auslösen können. Diese neue Klasse von Zytostatika ist aber besonders interessant, weil sie ein großes Potential in der Tumorbekämpfung hat und mit wesentlich weniger Nebenwirkungen verbunden ist als die klassischen Zytostatika.

Zur Identifikation von Substanzen, die DNA-schädigend wirken und daher potentiell als Zytostatika eingesetzt werden könnten, gibt es folgende verbreitete In-vitro-Testsysteme: Der Ames-Test oder auch Salmonella-typhimurium-Test (STY) basiert auf der Mutagenität von Substanzen in Bakterien, während der SOS-Chromotest auf der Induktion des bakteriellen SOS-Systems durch genotoxische Agenzien beruht. Beide Tests sind in ihrer Sensitivität vergleichbar, haben aber den grundsätzlichen Nachteil, daß die genotoxische Wirkung von Substanzen in Bakterien und höheren Organismen unterschiedlich sein kann.

Daher wurden der Micronucleus-Test, der Einzel-Zell-Geltest (SCG-Test), auch Kometen-Assay genannt, und der Test auf Schwester-Chromosomen-Austausch (SCE-Test) entwickelt, die auf eukaryontischen Zellsystemen basieren. In der Literatur ist eine Zellinie, A4/4, beschrieben, die ein lacZ-Gen unter der Kontrolle des Schwermetall-induzierbarem Metallothioneinpromotors enthält. Die Autoren beschreiben, daß der Promotor während der Kultivierung abgeschaltet wird, sich aber durch die demethylierende Substanz 5'Azacytidine wieder induzieren läßt {Biard et al. 1992}.

Die bisher bekannten Testsysteme zur Identifizierung DNAschädigender Agentien sind jedoch nicht geeignet, um Differenzierung auslösende Substanzen zu identifizieren, da der Mechanismus ein völlig anderer ist.

Die von Biard et al. (a.a.O) beschriebene Reporterzellinie hat den entscheidenden Nachteil, daß es sich um ein induzierbares System handelt. Die demethylierende Wirkung von Substanzen kann nur sichtbar gemacht werden, wenn gleichzeitig der Inducer für den Promotor verwendet wird. Die Autoren haben den Metallothioneinpromotor gewählt, der durch Schwermetalle wie Cadmium und Zink induziert wird. Es gibt hunderte von Hinweisen aus der Literatur, daß Schwermetalle ihrerseits Genexpression induzieren. Dadurch wird die demethylierende Wirkung einiger Substanzen durch die Genexpression-auslösende Wirkung der Schwermetalle überlagert, die für das System benötigt werden. Das heißt, unspezifische, falsch negative oder falsch positive Ergebnisse sind sehr leicht möglich. Die Zellinie wurde hergestellt mit dem Zweck, demethylierende Substanzen entdecken zu können. Diese Substanzen können Differenzierung auslösen, es sind aber - wie oben ausgeführt - nicht die einzigen Substanzen, die dazu in der Lage sind. Da es keine weiteren Publikationen mit dieser Zellinie nach 1992 gibt ist weder bekannt, ob diese Zellinie stabil ist, noch ob sie geeignet ist, auch andere Differenzierungsprozesse nachzuweisen.

Vor diesem Hintergrund liegt der Erfindung die Aufgabe zugrunde, ein Verfahren der eingangs erwähnten Art bereitzustellen, mit dem schnell, einfach und zuverlässig Differenzierung auslösende Substanzen identifiziert werden können, sowie in dem Verfahren verwendbare Hilfssubstanzen.

Diese Aufgabe wird gelöst durch ein DNA-Konstrukt, das ein Fusionsgen unter der Kontrolle eines Promotors enthält, wobei das Fusionsgen mindestens ein Resistenzgen und mindestens ein Reportergen umfaßt sowie für eine mit dem DNA-Konstrukt transfizierte Wirtszelle in geringem Maße toxisch ist, so daß der Promotor abgeschaltet wird, wenn eine Expression des Resistenzgens für das Wachstum der transfizierten Wirtszellen nicht erforderlich ist, da bspw. das entsprechende Antibiotikum in dem Nährmedium fehlt.

Ebenfalls beschrieben ist ein Plasmid mit einem derartigen DNA-Konstrukt, ein Virus mit einer Expressionskassette, die das DNA-Konstrukt beinhaltet oder dafür kodiert, also ein DNA-Virus oder ein Retrovirus, sowie eine eukaryontische Zelle, insbesondere humane Zelle, die stabil mit dem Plasmid tranfiziert oder dem Virus infiziert ist, insbesondere die Zellinie U87-HGFP, die am 09.11.2000 nach dem Budapester Vertrag bei der DSMZ in Braunschweig unter der Hinterlegungsnummer DSMZ ACC 2473 hinterlegt worden ist, sowie die Verwendung des DNA-Konstruktes und/oder des Plasmids und/oder des Virus und/oder der Zelle zur Identifizierung von Substanzen, die bei eukaryontischen Zellen eine Differenzierung auslösen.

Die Aufgabe wird ferner gelöst durch ein Verfahren zur Identifizierung von Substanzen, die bei eukaryontischen Zellen eine Differenzierung auslösen können, mit den Schritten:
a) Inkubieren der neuen Zellen in einem Kulturmedium mit einer dem Resistenzgen entsprechenden Selektionssubstanz,
b) Überimpfen der inkubierten Zellen aus Schritt a) in ein Kulturmedium, dem die Selektionssubstanz fehlt, und Inkubation der übergeimpften Zellen für ca. 5 bis ca. 100, vorzugsweise ca. 24 h,
c) Zugabe einer zu identifizierenden Substanz zu dem Kulturmedium der Zellen aus Schritt b) und weiter Inkubation für ca. 1 bis ca. 5, vorzugsweise ca. 2 Tage, und
d) Überprüfung der inkubierten Zellen aus Schritt c) auf verglichen mit Zellen aus Schritt b) erhöhte Expression des Reportergens.

Das Problem der Identifizierung Differenzierung auslösender Substanzen wird durch die Erfindung vollständig gelöst.

Dabei ist es bevorzugt, wenn der Promotor ausgewählt ist aus CMV-Promotor, RSV-Promotor, zellulären Promotoren von Tumorsuppressorgenen und Promotoren von Differenzierungsgenen, das Reportergen ausgewählt ist aus GFP, LacZ, Luciferase, das Resistenzgen ausgewählt ist aus Hygromycingen, Neomycingen, Puromycingen, und/oder das Fusionsgen zumindest ein Gen enthält, das für ein Polypeptid kodiert, das für die Wirtszelle eine toxische Wirkung entfalten kann, wie bspw. GFP, das selbst leicht toxisch ist, oder Cytosindeaminase oder Thymidinkinase, die die Prodrugs 5'Fluorcytosin bzw. Ganciclovir zu toxischen Substanzen umsetzen.

Die Zellinie umfaßt also ein Fusionsgen unter Kontrolle des humanen Cytomegalivirus Promotor (CMV-Promotor). Dieser Promotor ist normalerweise äußerst stark in Zellen und muß daher - anders als im Stand der Technik der Metallothioneinpromotor - nicht induziert werden. Es ist außerdem bekannt, daß der Promotor unter bestimmten Bedingungen abgeschaltet werden kann, insbesondere in vivo.

Das Fusionsgen besteht dabei aus dem Resistenzgen für das Antibiotikum Hygromycin (Hygro) und dem Green Fluorescence Protein (GFP). Die Transfektion der Glioblastomzellinie U87 mit diesem Konstrukt führte nach Selektion mit dem Antibiotikum Hygromycin zur Erzeugung der Zellinie U87-HGFP. Diese Zellinie exprimiert das Fusionsgen in Gegenwart des Antibiotikums, was in der Fluoreszenzmikroskopie sehr gut anhand der GFP-Fluoreszenz sichtbar ist. Wird das Antibiotikum nur für wenige Tage abgesetzt, reguliert die Zelle den CMV-Promotor herunter, da das Fusionsprotein etwas toxisch für die Zellen ist. Nach Beobachtungen der Erfinder reichert es sich in bestimmten Zellkompartimenten (wahrscheinlich das ER) an. Die Behandlung der Zellen mit den Differenzierung auslösenden Substanzen 5'-Azacytidin und/oder Trichostatin A in sehr geringen Konzentrationen führt innerhalb von nur zwei Tagen zu einer sehr starken Hochregulierung des CMV-Promotors, sichtbar durch gesteigerte GFP-Fluoreszenz.

Bisher war noch keine Zellinie beschrieben oder bekannt, in der Differenzierung auslösende Substanzen mit einer Expressionseinheit bestehend aus CMV-Promotor und Hygromycin-GFP Fusion nachgewiesen werden können. Das System ist sehr zuverlässig, die Anwesenheit von Hygromycin während der Routinekultivierung der Zellinie verhindert den Verlust der Expressionseinheit. Die Zellinie hat die intrinsische Fähigkeit, den CMV-Promotor nur wenige Tage nach Absetzen von Hygromycine fast vollständig herunterzuregulieren. Der Promotor wird wieder hochreguliert, wenn Differenzierungs-auslösende Substanzen gegeben werden.

Der Vorteil gegenüber dem bekannten System ist u.a. die Verwendung eines Fusiongens, das mehrere Eigenschaften vereint: (I) positive Selektierbarkeit durch das Hygromycingen, d.h. die Expressionseinheit bleibt stabil vorhanden, wenn das Antibiotikum gegeben wird; (II) negative Selektierbarkeit, d.h. durch den toxischen Effekt des GFP-Gens wird in Abwesenheit von Hygromycin B auf Zellen selektioniert, in denen der Promotor abgeschaltet wird; (III) Identifizierbarkeit durch die Eigenfluoreszenz des GFP. Durch diese Eigenschaften ist das System sehr stabil und zuverlässig.

Das System braucht keinen Inducer außer der zu untersuchenden Differenzierunssubstanz und ist daher unbeinflußt von Störungen bzw. Überlagerungen eines Inducers.

Das System ist nachweisbar in der Lage, nicht nur Demethylierung sondern auch Histonacetylierung zu entdecken.

Durch die Verwendung des GFP als Reporter ist es möglich, die Promotoraktivität und damit die Wirkung Differenzierung auslösender Substanzen sowohl in lebenden Zellen als auch in fixierten Zellen zu beobachten (Fluoreszenzmikroskopie) und mittels Durchflußzytometrie exakt und reproduzierbar zu quantifizieren.

Andere Reporterexpressionseinheiten, bestehend aus einem Promotor (CMV-Prom, RSV-Prom., zelluläre Promotoren von Tumorsuppressorgenen oder Differenzierungsgenen), einem Selektionsmarker (Hygromycingen, Neomycingen, Puromycingen etc.) fusioniert mit einem Reporter (GFP, LacZ, Luciferase) sowie einem "toxischen" Gen (z.B. GFP, Cytosindeaminase, HSV-Thymidinkinase) können bei dem neuen Verfahren ebenfalls eingesetz werden.

Bei der Cytosindeaminase oder Thymidinkinase müßten dann noch toxische aber nicht lethale Konzentrationen der Prodrugs 5'Fluorcytosin bzw. Ganciclovir gegeben werden. Dadurch würde auf Zellen selektioniert, die den Promotor herunterregulieren. Diese Konstrukte können auch in andere Zellenlinien als U87 transfiziert werden oder die Untersuchungen können auch in vivo, d.h. an transgenen Tieren vorgenommen werden.

Ein besonderer Vorteil bei der Zellinie U87-HGFP liegt darin, daß der Promotor in kurzer Zeit und zuverlässig abgeschaltet werden kann. Ferner ist das Verfahren automatisierbar, so daß viele Substanzen in kurzer Zeit gescreent werden können.

Weiter ist von Vorteil, daß es sich um eine Tumorzellinie handelt, so daß nach den Substanzen in einer Krebszelle gesucht wird, die damit nicht nur ein Modellsystem sondern zugleich ein Testsystem ist. Weiter ist von Vorteil, daß diese Zellen bei Zugabe der entsprechenden Substanzen differenzieren, was an der Veränderung der Morphologie erkannt werden kann.

Eine so identifizierte Substanz ist also nicht nur in der Lage, einen heruntergeregelten Promotor wieder zu aktivieren, sondern sie kann eine Krebszelle in die Differenzierung treiben und ist damit ein potentielles Zytostatikum.

Vor diesem Hintergrund ist auch die Verwendung einer mit dem neuen Verfahren identifizierten Substanz zur Herstellung einer pharmazeutischen Zusammensetzung zur Behandlung von malignen und benignen Tumorerkrankunge beschrieben, sowie ein Verfahren zur Herstellung einer pharmazeutischen Zusammensetzung, das das neue Verfahren sowie das Mischen der identifizierten Substanz mit einem pharmazeutisch verträglichen Träger umfaßt.

Weitere Vorteile ergeben sich aus der Beschreibung und der beigefügten Zeichnung.

Es versteht sich, daß die vorstehend genannten und nachstehend noch zu erläuternden Merkmale nicht nur in den jeweils angegebenen Kombinationen sondern auch in anderen Kombinationen oder in Alleinstellung verwendbar sind, ohne den Rahmen der vorliegenden Erfindung zu verlassen.

Die Erfindung wird jetzt anhand von Ausführungsbeispielen und der beigefügten Zeichnung erläutert. Es zeigen:

### Figur 1

Detektion von H-GFP Gen Verstärkung durch 5-Azacytidin und Trichostatin A mittels Fluoreszenzmikroskopie: U87 H-GFP Zellen, auf Deckgläsern wachsend, wurden für 48 h mit 5-Azacytidin (0,5-2,5-20 µM) und Trichostatin A (0,1-1 µM) behandelt. Aufgrund der Verstärkung des H-GFP Gens konnte beim Vergleich von unbehandelten mit behandelten Zellen ein ansteigendes grünes Fluoreszenz-Signal beobachtet werden. Die Elternzellen besaßen kein für die Beobachtung durch Fluoreszenz-Mikroskopie ausreichend starkes Signal (Daten nicht gezeigt). Alle Bilder entsprechen einer 400X-Vergrößerung.

### Figur 2

FACS Fluoreszenz-Profile, mit U87 H-GFP und Elternzellen nach 48 h Behandlung unter Verwendung von unterschiedlichen Konzentrationen an 5-Azacytidin und Trichostatin A erhalten: 1-2x10⁵ Zellen von U87 H-GFP und der Elternlinie wurden in 6-Loch-Platten ausgesät, für 48 h mit 5-Azacytidin und Trichostatin A behandelt und anschließend durch Durchflußzytometrie wie nachstehend beschrieben analysiert. In allen Histogrammen entsprechen die Abszissen einem willkürlichen und logarithmischen Maßstab, der sich auf die Fluoreszenz-Intensität bezieht, wohingegen sich die Ordinaten auf die Zellzahl beziehen. Jede der Kurven entspricht 2x10⁴ ausgezählten Zellen, und in den Überlagerungen entspricht das dunklere Profil den unbehandelten Zellen und das hellere den behandelten.

Profile (a) bis (e): U87 H-GFP. Eine Verschiebung zu höherer Fluoreszenz-Intensität ist eindeutig sichtbar mit 2,5, 20 µM Aza und 1 µM TSA, wohingegen 0,5 µM Aza und 100 nM TSA-Kurven die der unbehandelten völlig überlagern.

Profile (f) bis (1): U87. Eine geringe Verschiebung kann besonders bei der Verwendung von 20 µM Aza und 1 µM TSA beobachtet werden, die jedoch als nicht durch das Plasmid ausgelösten Hintergrundanstieg betrachtet und nicht durch Fluoreszenz-Mikroskopie beobachtet werden kann.

### Figur 3

(A) U87 Elternlinie und H-GFP, für 48 h mit unterschiedlichen Endkonzentrationen von 5-Azacytidin behandelt. U87 H-GFP zeigt einen zweifachen Anstieg im Mittelwert der Fluoreszenz-Intensität bereits bei 2,5 µM Aza (im Vergleich zu unbehandelten Zellen). Diese Reporter-Zellinie erreicht einen dreifachen Anstieg bei einer Verwendung von 20 µM Aza. Einen ungefähr zweifachen Anstieg im Mittelwert der Fluoreszenz-Intensität der U87 Eltern erhalten wir nur bei der höchsten verwendeten Konzentration.
(B) Anstieg der H-GFP Genexpression aufgrund von Trichostatin A. Ungefähr 105 Zellen jeder Zellinie wurden für 48 h mit unterschiedlichen Endkonzentrationen an Trichostatin A in Kultur gehalten. Wie man im Histogramm erkennen kann, erhielten wir bei Verwendung einer Endkonzentration von 1 µM und 3 µM TSA in U87 H-GFP im Mittelwert der Fluoreszenz-Intensität einen dreifachen und vierfachen Anstieg (im Vergleich zu unbehandelten).
   In beiden Histogrammen stammen alle Mittelwerte, Standardabweichungen und P-Werte von mindestens fünf unterschiedlichen Werten (n=5), erhalten aus zwei unterschiedlichen und unabhängigen Experimenten. Alle P-Werte beziehen sich auf unbehandelte Zellen.
(C oben) Behandlung mit zwei unterschiedlichen Kombinationen von 5-Azacytidin und Trichostatin A. Die zwei Zellinien (10⁵ Zellen/Vertiefung) wurden für 48 h in Gegenwart von zwei unterschiedlichen Kombinationen von Aza und TSA kultiviert. In beiden Fällen kann in U87 H-GFP ein dreifacher Anstieg im Mittelwert der Fluoreszenz-Intensität beobachtet werden (im Vergleich zu unbehandelten). Der Unterschied ist jedoch nicht signifikant (p = 0,09), wenn wir den Anstieg bei U87 H-GFP vergleichen, die mit den zwei unterschiedlichen Kombinationen der Medikamente behandelt wurden.

(C unten) Trichostatin A und 5-Azacytidin jeweils einzeln und in Kombination. 10⁵ Zellen der U87 H-GFP Zellinie wurden für 48 h mit 200 nM TSA, 1 µM Aza und mit der Kombination beider behandelt. Bei Verwendung der zwei Medikamente jeweils einzeln erhielten wir einen ungefähr zweifachen Anstieg im Mittelwert der Fluoreszenz-Intensität (P~10⁻⁹ und <10⁻⁴ im Vergleich zu unbehandelten). Wenn die Kombination beider Medikamente verwendet wurde, erhielten wir einen geringen und signifikanten Anstieg in der Fluoreszenz-Intensität im Vergleich zu den Medikamenten jeweils alleine (P~0,02).

Die Mittelwerte, Standardabweichungen und P-Werte basieren auf zehn Werten (n = 10), die in zwei unabhängigen Experimenten ermittelt wurden.

### Figur 4

Dot plots, durch FACS-Analyse von U87 H-GFP und der Elternlinie erhalten, die für 48 h mit 5 µM 5-Azacytidin behandelt und nicht behandelt wurden. Beide Zellinien wurden in Gegenwart von 5 µM Aza in Kultur gehalten. Zwei Tage später wurden die Zellzyklen wie beschrieben bestimmt. 10.000 Zellen wurden analysiert. Im folgenden wird die Vorgehensweise zur Untersuchung des Zellzyklus durch FACS-Analyse gezeigt.

[I und IV] Dot plots, auf DNA-Färbung behandelter und unbehandelter Zellen bezogen. Um eine (2N+4N) Nuclei-Population genau zu definieren, wurde eine Region (R1) benutzt. [II und V] Dot plots, auf den BrdU-Einbau in DNA bezogen. Die Werte 200 und 400 auf dem linearen Maßstab der FL3-A Fluoreszenz entsprechen der 2N und 4N DNA-Menge in den Nuclei. Alle gezeigten Ereignisse entsprechen der R1-Region. [III und VI] Isotypenkontrolle, zur Festlegung des Quadranten für die Dot plots des BrdU-Einbaus verwendet. Der Anti-Isotyp-Antikörper gibt die unspezifische Bindung wieder und wurde als Negativ-Kontrolle für den Anti-BrdU-Antikörper verwendet. Die Prozentsätze von Zellen in unterschiedlichen Phasen des Zellzyklus wurden unter Verwendung der Dot plots des BrdU-Einbaus bestimmt (im Quadranten: Untere Linke Region = G1-G0; Untere Rechte Region = G2; Obere Linke Region = S; Obere Rechte Region = M).

### Figur 5

Das in den Experimenten verwendete Plasmid pCMV-HygroEGFP.

### 1) Klonierung von pCMV-HygroEGFP

Intermediates Plasmid 1 mit Namen pScriptpolyA (3595 bp):

Ausgangsplasmid pCRScript SK(+) AmpR+ geschnitten mit EcoRV und HindIII, und anschliessender Ligation mit SmaI/HindIII Fragment, das ein 625 bp HSV-Thymidinkinase-polyadenylierungssignal aus pTKneo enthält (blunt end ligation)

### Intermediat 2: pCMVA (4260 bp)

Insertion eines 670 bp hCMV Promotorfragments aus [pL15Tk geschnitten mit PstI, Enden mit T4 DNA Polymerase geglättet] in das Intermediat 1, pScriptpolyA, das mit SrfI geschnitten wurde (blunt end ligation).

### Intermediat 3: pCMV-EGFP (4975 bp)

Einligieren des EGFP Leserahmen aus Plasmid pEGFP (Clontech), das mit BamHI/ NotI geschnitten wurde (Enden mit Klenow Polymerase aufgefüllt) in den mit PstI geöffneten Vektor pCMVA (Intermediat 2, Enden mit T4 DNA Polymerase geglättet) (blunt end ligation).

### Fertiges pCMV-HygroEGFP (6052 bp)

Einligierung eines 1026 bp PCR Fragmentes mit dem offenen Leserahmen des Hygromycinresistenzgenes aus pTkHygro (Stopcodon durch die PCR entfernt) in den mit AgeI geöffneten Vektor pCMV-EGFP (Enden mit Klenow Polymerase aufgefüllt und so geglättet) (blunt end ligation).

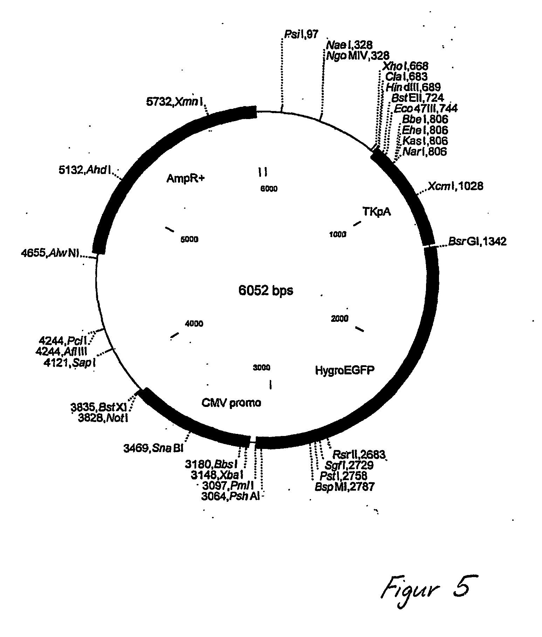

Das Plasmid ist in Fig. 5 gezeigt.

### 2) Material und Methoden

### Zellinien

U87 H-GFP ist eine von der humanen Glioblastom-Zellinie U87 nach stabiler Transfektion mit dem Plasmid pCMV-hygroEGFP abgeleitete Zellinie. Dieses in die genomische DNA integrierte Plasmid entstammt dem PCR-Script^{™} (Stratagene) und trägt stromabwärts des humanen CMV-Promoters ein Fusionsgen. Dieses Fusionsgen enthält das Gen für die Resistenz gegen Hygromycin verbunden mit dem Gen, das für das verstärkt grün fluoreszierende Protein (EGFP) kodiert. Das resultierende Gen (H-GFP genannt) kodiert für ein Protein, das die Resistenz gegenüber Hygromycin B verleiht und das durch Fluoreszenz-Mikroskopie und Durchflußzytometrieanalyse einfach detektiert werden kann. Im Gegensatz dazu hat die Elternzellinie U87 innen kein Plasmid und kann als Negativ-Kontrolle verwendet werden.

U87 H-GFP und die Elternlinie U87 wurden in Dulbecco's Modifiziertem Eagle's Medium (D-MEM, niedriger Glukoseanteil, Gibco, BRL) ergänzt mit 10 % fötalem Kälberserum (Seromed), 100 Units/ml Penizillin, 100 µg/ml Streptomycin und 1 µg/ml Amphotericin B (Gibco, BRL) bei 37°C in befeuchteter Atmosphäre mit 5% CO₂-Anteil kultiviert. Für die U87 H-GFP Zellinie wurde das Kulturmedium mit 600 Units/ml Hygromycin B (Calbiochem) ergänzt.

U87 H-GFP und die Elternzellen wurden mit 1-2 x 10⁵ Zellen/Vertiefung auf 6-Loch-Platten (Nuclon, NUNC) unter Verwendung von D-MEM ohne Hygromycin B ausgesät und zur Anheftung für 24 h inkubiert. Daraufhin wurden 5-Azacytidin und Trichostatin A (Sigma Chemicals, Co.) in unterschiedlichen Endkonzentrationen (2,5-5-10-20-40 µM bei Aza; von 10 nM bis 3 µM für TSA) zum Kulturmedium hinzugefügt. Fluoreszenz-Mikroskopie und Durchflußzytometrie-Analysen wurden nach zwei Behandlungstagen durchgeführt.

### Fluoreszenz-Mikroskopie

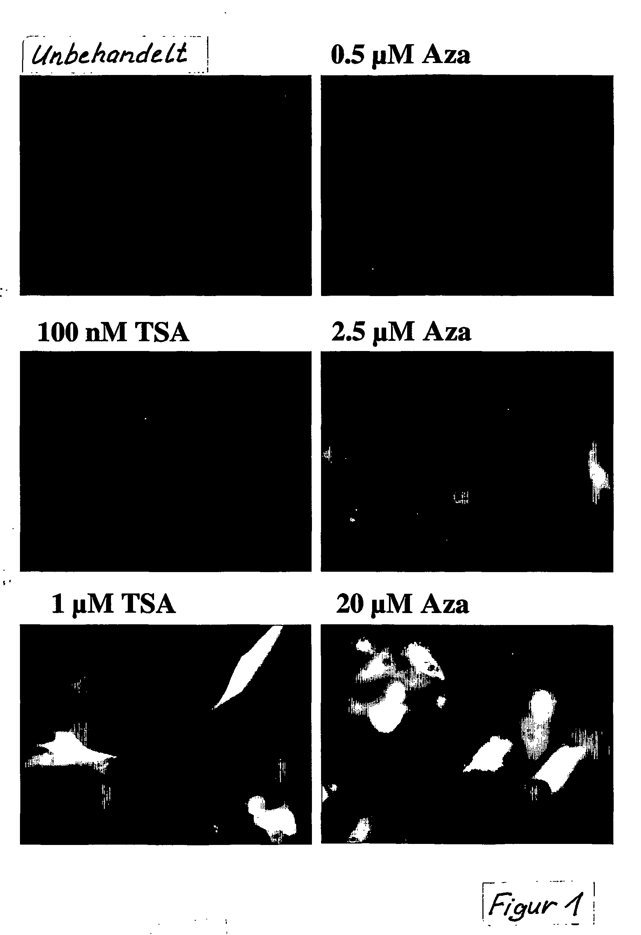

Ungefähr 10⁵ Zellen jeder Zellinie wurden auf mit Polylysin beschichtete Deckgläser ausgesät, mit unterschiedlichen Konzentrationen an Aza und TSA für 48 h behandelt und daraufhin in 5% Formaldehyd für 30 min bei Raumtemperatur fixiert. Danach wurden alle Proben unter dem Fluoreszenz-Mikroskop (Axiophot, Zeiss, Deutschland) analysiert und verschiedene Bilder aufgenommen, die den unterschiedlichen Endkonzentrationen jedes Medikamentes entsprechen (Fig. 1).

Durchflußzytometrie-Analysen von Zellen, die das Hygromycin-EGFP-Fusionsgen exprimieren.

Um Proben für die FACS-Analyse vorzubereiten, wurden Zellen in den 6-Loch-Platten in 0,05% Trypsin, das 0,5 mM EDTA (Gibco, BRL) enthielt, für 5 min bei 37°C inkubiert, und daraufhin wurde die Wirkung des Trypsins durch Hinzufügen des zweifachen Volumens des Kulturmediums gestoppt. Die Zellen wurden geerntet, für 5 min bei 500xg zentrifugiert und in 1 ml komplettiertes D-MEM resuspendiert. Um tote Zellen anzufärben, wurde zu jeder Probe Propidiumiodid (Endkonzentration 10 µg/ml) hinzugegeben. Daraufhin wurden die Zellen wieder abzentrifugiert und in mit Phosphat gepufferter Kochsalzlösung (PBS 1X, pH 7,4) resuspendiert, bevor sie mittels FACS analysiert wurden.

Die Zellen wurden mit FACScalibur (Becton Dickinson) analysiert, die Durchflußrate entsprach ungefähr 500 Ereignissen/s.

Um eine Zellpopulation genau zu definieren und um Zelltrümmer und Aggregate auszuschließen, wurde auf dem Dot plot (FSC im Vergleich zu SSC) ein interessierender Bereich festgelegt. Auf dem Biparameter-Histogramm von Propidiumiodid wurden tote Zellen durch Vergleich zum "Forward angle light scatter" (FSC) unterschieden. Die Fluoreszenz-Intensität einzelner Zellen wurde auf einem logarithmischen Maßstab gemessen, wobei jedes logarithmische Histogramm 2 x 10⁴ ausgezählte Ereignisse darstellt. Der Mittelwert der Fluoreszenz-Intensität (M.F.I.) war der Parameter, der für die Definition des Anstiegs in der Fluoreszenz-Intensität in jeder Zellpopulation benutzt wurde.

### Zellzyklus-Analyse

U87 H-GFP und die Elternzellen wurden mit 5x10³/cm² in T25-Flaschen (Nuclon, NUNC) unter Verwendung von D-MEM für beide Zellinien ohne Hygromycin B ausgesät. Eine Hälfte der Flaschen wurde mit 5 µM Aza und die andere als Negativkontrolle ohne jegliche Medikamente behandelt. Nach 42 h wurde dem Medium BrdU hinzugefügt (Endkonzentration von 10 µM) und 6 h später wurden die Zellen für die Zellzyklus-Analyse vorbereitet.

Die Zellen wurden geerntet, für 5 min bei 500xg zentrifugiert und für 20 min mit gekühltem 70%igem Ethanol fixiert. Für die Detektion des BrdU-Einbaus in die DNA wurden 3 x 10⁵ Zellen für jede mit Aza behandelte oder nicht behandelte Zellinie verwendet.

PBS 1X/ 0,5% BSA (Waschpuffer) wurden hinzugefügt, und die Zellen wurden für 5 min bei 500xg zentrifugiert. Daraufhin wurde das Pellet in einer denaturierenden Lösung (HCl 1M, PBS 0,5X, BSA 0,5%) resuspendiert und nach 20 min wieder gewaschen. Anschließend wurde das Pellet in 0,1M Natriumborat (Na₂B₄O₇) pH 8,5, für 2 min resuspendiert, und daraufhin der Waschpuffer hinzugefügt. Nach dieser Passage wurde das Gesamtvolumen jeder Probe in zwei Hälften aufgeteilt (eine Hälfte wurde für den Isotyp-Ak, die andere für den BrdU-Ak verwendet), für 5 min bei 500xg zentrifugiert, in dem Puffer, der die Anti-Isotyp- oder die Anti-BrdU monoklonalen Antikörper (Becton Dickinson) enthielt, resuspendiert und nach 30 min mit PBS 1X/0,5% BSA gewaschen. Der Überstand wurde verworfen und das Pellet in RNAse A (Endkonzentration 100 µg/ml) resuspendiert; um die DNA zu färben, wurde 7-AAD (Via-PROBE, Becton Dickinson) ebenfalls zu der Lösung hinzugegeben. Nach 1 h (im Dunkeln) wurden die Proben gewaschen und danach in PBS 1X/0,5% BSA resuspendiert. Die DNA-Fluoreszenz der Nuclei (für jede Zellpopulation wurden ungefähr 10⁴ Nuclei analysiert) wurde mittels des zuvor erwähnten FACScan-Durchflußzytometers (Becton Dickinson) gemessen, und die Prozentsätze von Zellen in den G0 und G1, S, G2 und M Phasen des Zellzyklus wurden anhand der FACScan-Softwareprogramme analysiert.

### 3) ERGEBNIS

Die Zellinie U87 H-GFP enthält das Plasmid pCMV-hygroEGFP, in welchem die Aktivierung des hCMV-Promotors normalerweise auf einer niedrigen Stufe reguliert wird. Es verleiht der Zellinie die Resistenz gegenüber Hygromycin B und eine grundlegende grüne Fluoreszenz im Vergleich mit der Elternzellinie. Es wurde gezeigt, daß der hCMV-Promotor durch Methylierung des Cytosins in der 5'-CpG-Stelle (Prosh S. et al., Biol Chem Hoppe Seyler 1996, Mar 377(3): 195-201) vollständig reprimiert wird. Um die Eigenschaft des Promotors abzuschätzen, durch Medikamente aktiviert werden zu können, die mit dem Methylierungsstatus der DNA und der Chromatinkondensation interferieren, wurden U87 H-GFP und die Elternlinie U87 für mehrere Tage in Gegenwart von Aza und TSA gehalten. Vor dem Beginn des Experiments wurde Hygromycin B aus dem Medium entfernt, um eine Abnahme in der grundsätzlichen Aktivierung des hCMV-Promotors zu erzielen und um weiterhin eine Promotor-Aktivierung durch Aza und TSA zu detektieren.

Fig. 1 zeigt Fluoreszenz-Mikroskopie-Aufnahmen der U87 H-GFP-Zellen, die für 48 h mit unterschiedlichen Endkonzentrationen an Aza (0,5-2,5-20 µM) und TSA (100nM, 1 µM) behandelt wurden. Durch Vergleich der grundsätzlichen Expression der Reporter-Zellinie (unbehandelte Zellen) mit den Proben, die mit unterschiedlichen Endkonzentrationen an Aza und TSA behandelt wurden, kann man eine Verstärkung der H-GFP-Genexpression in den Bildern erkennen. Der Anstieg des grün fluoreszierenden Signals ist bei 20 µM Aza und 1 µM TSA deutlich definiert. Elternzellen zeigen einen schwachen Anstieg in der grundsätzlichen grünen Fluoreszenz, was nur durch FACS-Analyse und nicht durch Fluoreszenz-Mikroskopie detektiert werden konnte.

Ferner konnte eine Veränderung in der Morphologie der Zellen bei den hohen Konzentrationen von TSA und Aza beobachtet werden, was darauf hinweist, daß diese Substanzen die Tumorzellen U87 wieder in die Differenzierung treiben können.

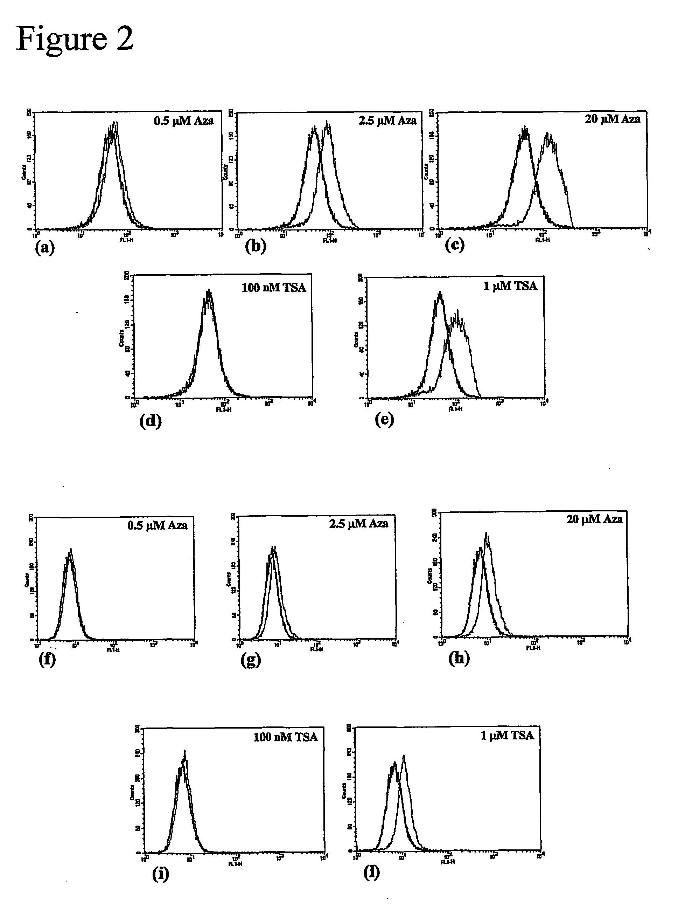

In Fig. 2 sind FACS Profile (logarithmische Histogramme) von Proben dargestellt, die mit den gleichen Konzentrationen an Aza und TSA wie bei der Fluoreszenz-Mikroskopie behandelt wurden.

Die Abszissen entsprechen einem willkürlichen Maßstab, der den Logarithmus der Fluoreszenz-Intensität bezeichnet, und die Ordinaten stellen die relative Zellzahl dar. Beim Vergleich der Kontrolle, die nicht mit der Reporterzellinie behandelt wurde, mit der Reporterzellinie, die mit den oben erwähnten Endkonzentrationen behandelt wurde, erhielten wir eine Verschiebung im Fluoreszenz-Profil in Richtung höherer Intensität. Die Verschiebung ist bereits eindeutig bei Aza-Zugaben von 2,5 µM (Fig. 2, Profil b) zu erkennen und ist bei höheren Konzentrationen gut definiert (Profil c).

TSA induziert in keiner Weise einen Anstieg in der H-GFP-Genexpression bei einer Endkonzentration von 100 nM (Profil d), erreicht aber eine Sättigungsstufe bei einer Endkonzentration von 1 µM (Profil 1). Endkonzentrationen von TSA, die höher als 3 µM lagen, konnten nicht verwendet werden, da sie sich als hoch giftig erwiesen und die Anzahl der lebenden Zellen drastisch reduzierten, was mittels FACS beobachtet werden konnte (Daten nicht gezeigt).

Die Elternzellinie ist ebenfalls sensitiv gegenüber Aza (Fig. 2, Profile f bis h) und TSA (Profil i und 1), die Verschiebung in der Fluoreszenz-Intensität ist jedoch nicht vergleichbar mit denen, die mit der Reporter-Zellinie erhalten wurde, und kann daher als Hintergrund-Anstieg, und nicht als durch das Plasmid hervorgerufen, erklärt werden.

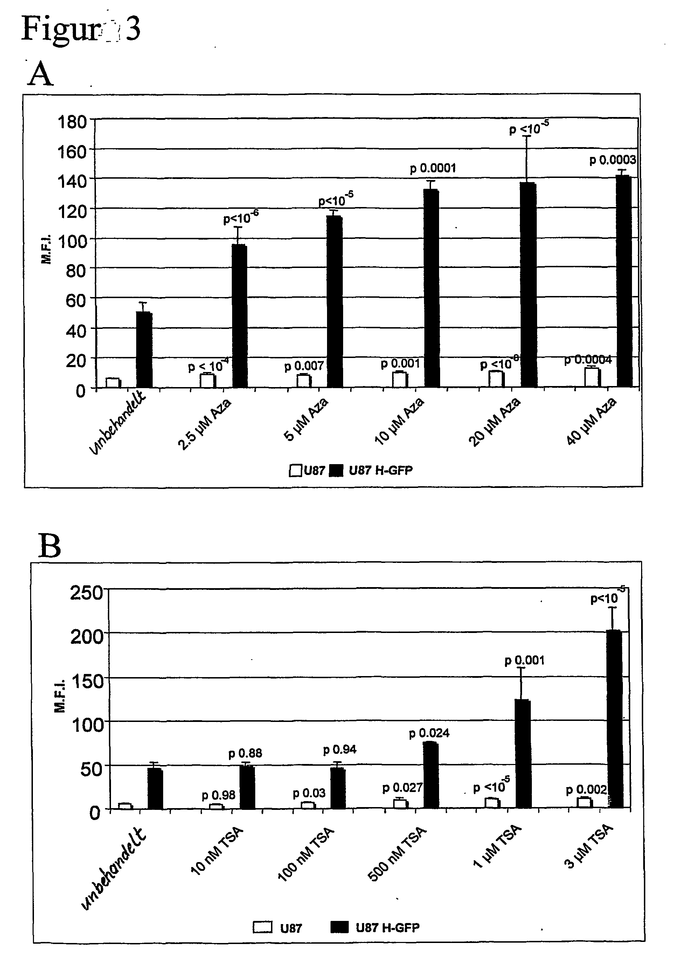

Das Histogramm in Fig. 3A entspricht dem Versuch, bei dem die Reporter-Zellinie und die Elternzellinie mit unterschiedlichen Konzentrationen an Aza für nur 48 h behandelt wurden. Wenn man die Kontrolle (U87 H-GFP unbehandelt) mit Zellen, die mit 2,5 µM Aza behandelt wurden, vergleicht (P < 10-6 im Vergleich zur Kontrolle), zeigt das Histogramm einen zweifachen Anstieg im Mittelwert der Fluoreszenz-Intensität. Die H-GFP-Genexpression kann bei Verwendung von 20 µM Aza einen dreifachen Anstieg in der Fluoreszenz-Intensität erreichen (P < 10-5 im Vergleich zur Kontrolle). Ein geringer Anstieg in der grundsätzlichen Fluoreszenz (nur mittels FACS-Analyse und nicht in der Fluoreszenz-Mikroskopie) ist ebenfalls in der Elternzellinie sichtbar (untere Balken im Histogramm), was weniger als dem Zweifachen bei Verwendung der höchsten Konzentration (40 µM Aza) entspricht. Auch dieser Versuch wurde unter Beibehaltung von Aza für 144 h (fünf Tage) und bei gleichen Endkonzentrationen wiederholt. Wir erhielten die gleiche Verschiebung im Mittelwert der Fluoreszenz-Intensität (Daten nicht gezeigt).

Auch TSA kann eine Verstärkung des H-GFP-Gens auslösen, wie man in Fig. 3B sehen kann. Beide Zellinien wurden mit unterschiedlichen Endkonzentrationen an TSA für 48 h in Kultur gehalten. Im Histogramm kann ein schwacher, aber signifikanter Anstieg in der Fluoreszenz-Intensität von U87 H-GFP bei TSA nur mit 500 nM und nicht mit den verwendeten geringeren Konzentrationen beobachtet werden. Wenn wir jedoch 1 µM und 3 µM TSA verwendeten, haben wir einen dreifachen und vierfachen Anstieg in der Fluoreszenz beobachtet (P = 0,001 und P < 10-5, im Vergleich zu unbehandelten). In der Elternzellinie ist ein zweifacher Anstieg nur mit 3 µM TSA zu erkennen.

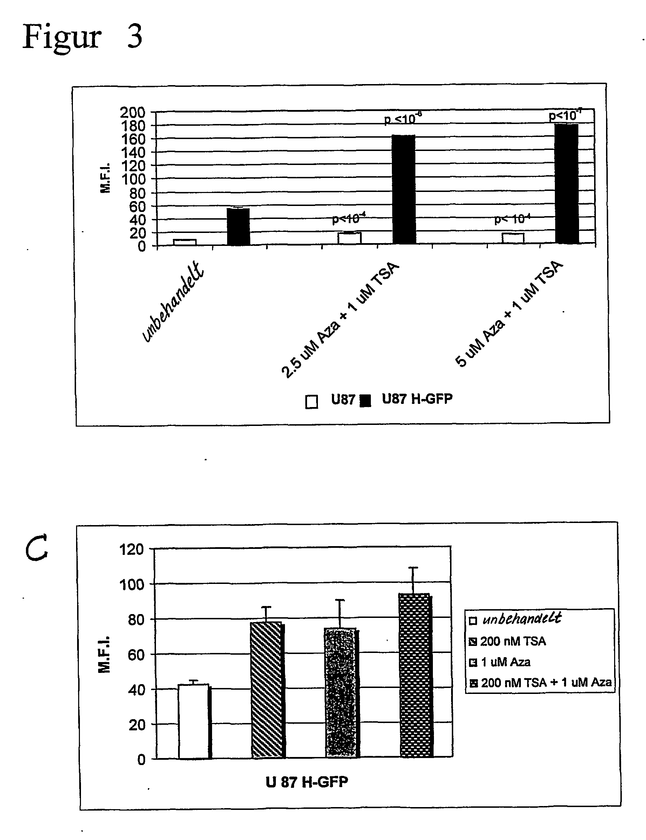

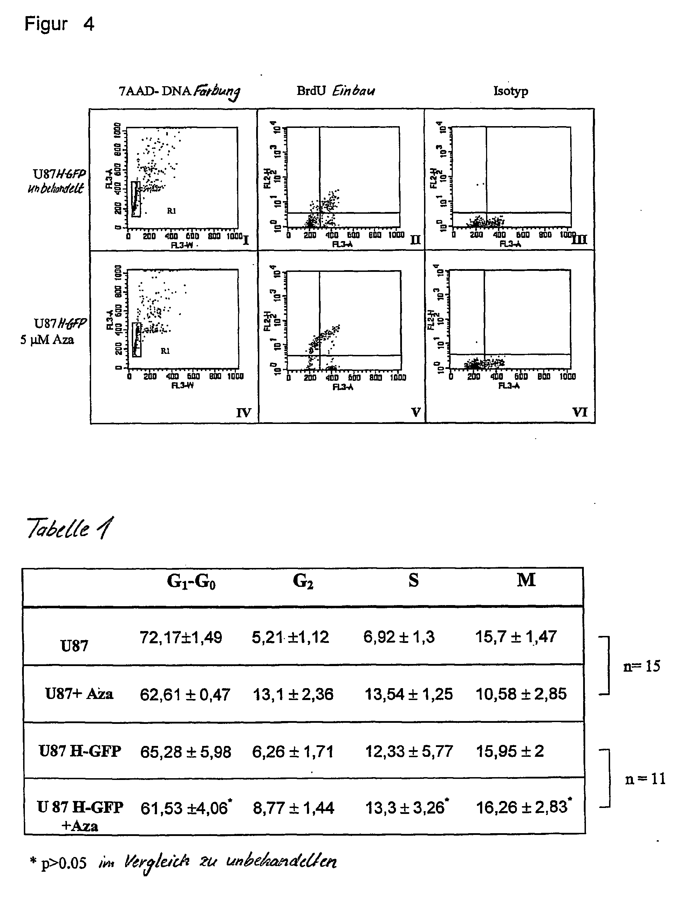

Um weiter zu untersuchen, ob TSA synergistisch mit Aza wirken kann, wie es bei anderen Tumorzellinien gezeigt wurde (Cameron E.E. et al., Nat. Genet. 1999; Bd 21, 103-107), verwendeten wir für 48 h TSA in Kombination mit Aza (Fig. 4c). In diesem Versuch hielten wir fixiertes TSA auf einer Endkonzentration von 1 uM, während wir Aza von 2,5 auf 5 µM veränderten. In beiden Fällen kann ein dreifacher Fluoreszenz-Anstieg in U87 H-GFP im Vergleich zu unbehandelten Zellen beobachtet werden, jedoch ist die Differenz nicht signifikant, wenn wir die beiden Kombinationen der Medikamente vergleichen (p = 0,09). Im folgenden Versuch (Fig. 4d) verwendeten wir für 48 h TSA und Aza jeweils alleine und in Kombination (Fig. 3c). Der Anstieg aufgrund der Medikamente alleine war signifikant und entsprach etwa einem zweifachen Anstieg sowohl mit 200 nM TSA (P ~ 10-9 im Vergleich zu unbehandelten) als auch mit 1 µM Aza (P < 10-4 im Vergleich zu unbehandelten). Wenn wir die Kombination beider benutzten (200 nM TSA + 1 µM Aza), war der geringe Anstieg in bezug auf die Medikamente alleine signifikant (P ~ 0,02 im Vergleich zu den Medikamenten alleine), jedoch schienen die Medikamente weder auf eine synergistische noch auf eine additive Weise zu wirken, was wahrscheinlich daran lag, daß der Anstieg in der Fluoreszenz-Intensität einer maximalen Aktivierung des CMV-Promotors mit diesen Medikament-Konzentrationen entsprach.

Effekte von 5-Azacytidin auf den Zellzyklus. U87 H-GFP und die Elternzellen wurden für zwei Tage mit 5 µM Aza behandelt, um den Effekt dieses Cytosin-Analogons auf dem Zellzyklus zu untersuchen. Wie man in Fig. 4 sehen kann, wurde die DNA mit 7-AAD gefärbt und in den ersten zwei Dot plots (I-IV) wurde ein Fenster benutzt (R1), um nur eine 2N und eine 4N Nuclei-Population genau zu definieren (entspricht den Werten 200 und 400 des FL3-A-Maßstabs). Die umschlossene Nuclei-Population wurde dann auf den BrdU-Einbau (II-V) unter Verwendung eines PE-konjugierten Antikörpers gegen BrdU untersucht. Die Isotypen-Färbung (Dot plots III-VI) wurde verwendet, um die unspezifische Bindung des BrdU-Antikörpers zu definieren und um den Quadranten in den BrdU-Einbau-Dot plots festzusetzen. Die Prozentsätze der Zellen in unterschiedlichen Zellzyklusphasen wurden durch die Dot plots des BrdU-Einbaus erhalten. Tabelle 1 zeigt die Prozentsätze an Zellen in unterschiedlichen Zellzyklusphasen. Azacytidin (in einer Konzentration von 5 µM) hat keinen signifikanten Effekt auf unterschiedliche Phasen von Zellen, mit Ausnahme einer kleinen Zunahme im Prozentsatz an Zellen in G2. Im Gegensatz dazu schien die Elternlinie (U87) gegenüber 5-Azacytidin sensitiver zu sein. In dieser Zellinie führte 5-Azacytidin zu einer verringerten Anzahl an Zellen in der M-Phase (~15,7 % in der Kontrolle im Vergleich mit ~10,6 % mit 5 µM Aza) und der G1-G0-Phase (~72,2 % in der Kontrolle im Vergleich zu ~62,6 % mit 5 µM Aza), verbunden mit einer gesteigerten Anzahl in der S-Phase (~6,9 % in der Kontrolle im Vergleich zu ~13,5 % mit 5 µM Aza) und der G2-Phase (~5,2 % in der Kontrolle im Vergleich zu ~13,1 % mit 5 µM Aza).

### 4) Statistische Analyse

Die P-Werte wurden unter Verwendung des Programms "Anova, 1-faktorielle-Varianz-Analyse" unter MS Excel berechnet. P-Werte < 0,05 wurden als statistisch signifikant erachtet.

Tabelle 1 zeigt die Zellzyklus-Analyse: 5-Azacytidin beeinflußt den Zellzyklus nur in der Elternzellinie U87, aber nicht in U87 H-GFP. Prozentsätze der Zellen in G1-G0-, S-, G2- und M-Phase des Zellzyklus wurden wie in Fig. 4 beschrieben erhalten.

U87 H-GFP zeigte bei Behandlung mit 5 µM Aza, abgesehen von einer erhöhten Anzahl von Zellen in der G2-Phase, keine signifikanten Unterschiede im Zellzyklus. Im Gegensatz dazu hatte U87 signifikante Änderungen in den Zellzyklus-Phasen, wenn sie für zwei Tage mit 5 µM Aza in Kultur gehalten wurde. Eine verringerte Anzahl an Zellen in den G1-G0- und M-Phasen war mit einer erhöhten Anzahl in den G2- und S-Phasen verbunden.

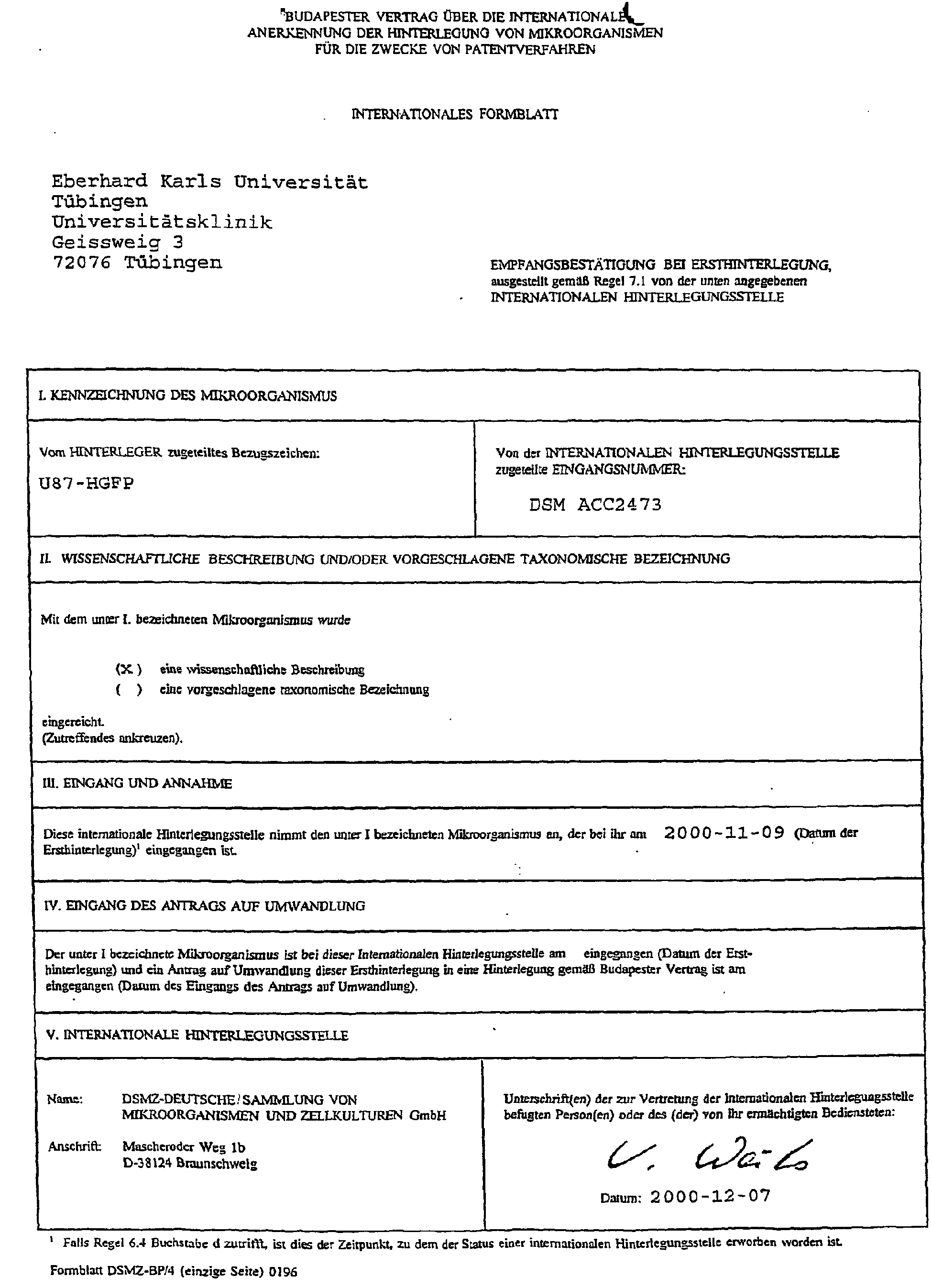
BUDAPESTER VERTRAG ÜBER DIE INTERNATIONALE
ANERKENNUNG DER HINTERLEGUNG VON MIKROORGANISMEN
FÜR DIE ZWECKE VON PATENTVERFAHREN

INTERNATIONALES FORMBLATT

Eberhard Karls Universität
Tübingen
Universitätsklinik
Geissweig 3
72076 Tübingen

EMPFANGSBESTÄTIGUNG BEI ERSTHINTERLEGUNG,
ausgestellt gemäß Regel 7.1 von der unten angegebenen
INTERNATIONALEN HINTERLEGUNGSSTELLE

I. KENNZEICHNUNG DES MIKROORGANISMUS
Vom HINTERLEGER zugeteiltes Bezugszeichen:
U87-HGFP | Von der INTERNATIONALEN HINTERLEGUNGSSTELLE
zugeteilte EINGANGSNUMMER:
DSM ACC2473

II. WISSENSCHAFTLICHE BESCHREIBUNG UND/ODER VORGESCHLAGENE TAXONOMISCHE BEZEICHNUNG

Mit dem unter I. bezeichneten Mikroorganismus wurde

(X) eine wissenschaftliche Beschreibung
( ) eine vorgeschlagene taxonomische Bezeichnung

eingereicht.
(Zutreffendes ankreuzen).

III. EINGANG UND ANNAHME

Diese internationale Hinterlegungsstelle nimmt den unter I bezeichneten Mikroorganismus an, der bei ihr am 2000-11-09 (Datum der
Ersthinterlegung)¹ eingegangen ist.

IV. EINGANG DES ANTRAGS AUF UMWANDLUNG

Der unter I bezeichnete Mikroorganismus ist bei dieser Internationalen Hinterlegungsstelle am eingegangen (Datum der Erst-
hinterlegung) und ein Antrag auf Umwandlung dieser Ersthinterlegung in eine Hinterlegung gemäß Budapester Vertrag ist am
eingegangen (Datum des Eingangs des Antrags auf Umwandlung).

V. INTERNATIONALE HINTERLEGUNGSSTELLE

Name: DSMZ-DEUTSCHE SAMMLUNG VON
MIKROORGANISMEN UND ZELLKULTUREN GmbH

Anschrift: Mascheroder Weg 1b
D-38124 Braunschweig | Unterschrift(en) der zur Vertretung der Internationalen Hinterlegungsstelle
befugten Person(en) oder des (der) von ihr ermächtigten Bediensteten:


Datum: 2000-12-07

¹ Falls Regel 6.4 Buchstabe d zutrifft, ist dies der Zeitpunkt, zu dem der Status einer internationalen Hinterlegungsstelle erworben worden ist.

Formblatt DSMZ-BP/4 (einzige Seite) 0196

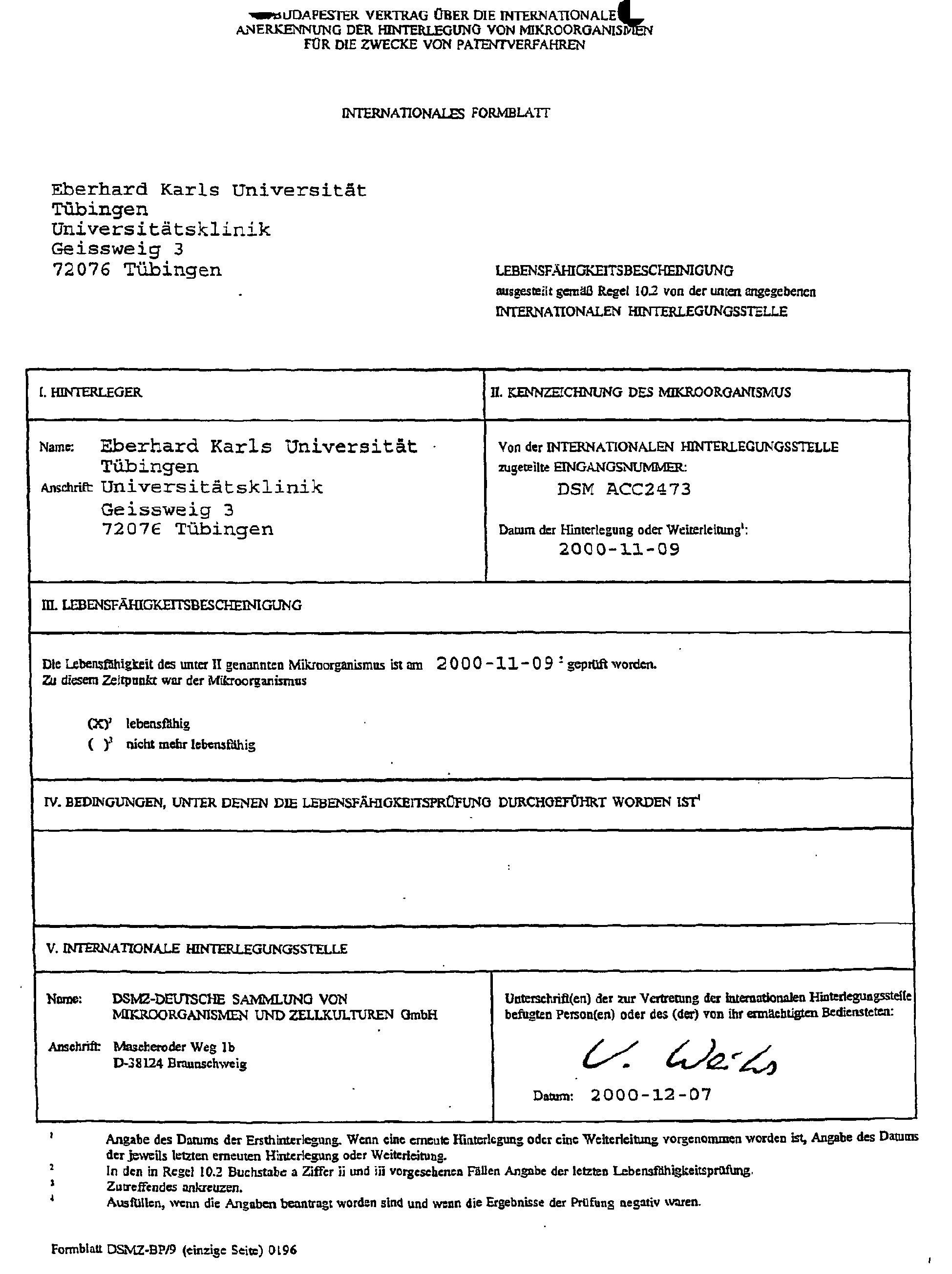
BUDAPESTER VERTRAG ÜBER DIE INTERNATIONALE
ANERKENNUNG DER HINTERLEGUNG VON MIKROORGANISMEN
FÜR DIE ZWECKE VON PATENTVERFAHREN

INTERNATIONALES FORMBLATT

Eberhard Karls Universität
Tübingen
Universitätsklinik
Geissweig 3
72076 Tübingen

LEBENSFÄHIGKEITSBESCHEINIGUNG
ausgestellt gemäß Regel 10.2 von der unten angegebenen
INTERNATIONALEN HINTERLEGUNGSSTELLE

I. HINTERLEGER | II. KENNZEICHNUNG DES MIKROORGANISMUS
Name: Eberhard Karls Universität
Tübingen
Anschrift: Universitätsklinik
Geissweig 3
72076 Tübingen | Von der INTERNATIONALEN HINTERLEGUNGSSTELLE
zugeteilte EINGANGSNUMMER:
DSM ACC2473
Datum der Hinterlegung oder Weiterleitung¹:
2000-11-09

III. LEBENSFÄHIGKEITSBESCHEINIGUNG

Die Lebensfähigkeit des unter II genannten Mikroorganismus ist am 2000-11-09 ² geprüft worden.
Zu diesem Zeitpunkt war der Mikroorganismus

(X)³ lebensfähig
( )³ nicht mehr lebensfähig

IV. BEDINGUNGEN, UNTER DENEN DIE LEBENSFÄHIGKEITSPRÜFUNG DURCHGEFÜHRT WORDEN IST⁴

V. INTERNATIONALE HINTERLEGUNGSSTELLE

Name: DSMZ-DEUTSCHE SAMMLUNG VON
MIKROORGANISMEN UND ZELLKULTUREN GmbH

Anschrift: Mascheroder Weg 1b
D-38124 Braunschweig | Unterschrift(en) der zur Vertretung der internationalen Hinterlegungsstelle
befugten Person(en) oder des (der) von ihr ermächtigten Bediensteten:


Datum: 2000-12-07

¹ Angabe des Datums der Ersthinterlegung. Wenn eine erneute Hinterlegung oder eine Weiterleitung vorgenommen worden ist, Angabe des Datums
der jeweils letzten erneuten Hinterlegung oder Weiterleitung.
² In den in Regel 10.2 Buchstabe a Ziffer ii und iii vorgesehenen Fällen Angabe der letzten Lebensfähigkeitsprüfung.
³ Zutreffendes ankreuzen.
⁴ Ausfüllen, wenn die Angaben beantragt worden sind und wenn die Ergebnisse der Prüfung negativ waren.

Formblatt DSMZ-BP/9 (einzige Seite) 0196

## Patentansprüche

1. Verwendung eines DNA-Konstruktes,
das ein Fusionsgen unter der Kontrolle eines Promotors enthält,
wobei das Fusionsgen mindestens ein Resistenzgen und mindestens ein Reportergen sowie zumindest ein Gen enthält, das für ein Polypeptid kodiert,
das für die transfizierte Wirtszelle eine in geringem Masse toxische Wirkung entfaltet
zur Identifizierung von Substanzen in einem Testsystem, die bei eukaryontischen Zellen eine Hypo- oder Demethylierung oder Histonacetylierung auslösen.

2. Verwendung eines DNA- Konstruktes nach Anspruch 1, **dadurch gekennzeichnet, dass** der Promotor ausgewählt ist aus CMV-Promotor, RSV-Promotor, zellulären Promotoren von Tumorsuppressorgenen und Promotoren von Differenzierungsgenen.

3. Verwendung eines DNA- Konstruktes nach Anspruch 1 oder 2, **dadurch gekennzeichnet, dass** das Reportergen ausgewählt ist aus GFP, LacZ, Luciferase.

4. Verwendung eines DNA-Konstruktes nach einem der Ansprüche 1 bis 3, **dadurch gekennzeichnet, dass** das Gen, das für ein Polypeptid kodiert, das für die transfizierte Wirtszelle eine toxische Wirkung entfaltet, ausgewählt ist aus GFP, Cytosindeaminase, HSV-Thymidinkinase.

5. Verwendung eines DNA-Konstruktes nach einem der Anprüche 1 bis 4, **dadurch gekennzeichnet, dass** das Resistenzgen ausgewählt ist aus Hygromycinresistenzgen, Neomycinphosphottransferasegen, Puromycin-N-acetyltransferasegen.

6. Verwendung eines DNA-Konstruktes nach einem der Ansprüche 1 bis 5, **dadurch gekennzeichnet, dass** das DNA-Konstrukt in einem Plasmid oder Virus enthalten ist.

7. Verwendung eines DNA-Konstruktes nach einem der Ansprüche 1 bis 5, **dadurch gekennzeichnet, dass** die eukaryontische Zelle ausgewählt ist aus einer humanen Zelle insbesondere Zelllinie U87-HGFP, die am 09.11.2000 nach dem Budapester Vertrag bei der DSMZ in Braunschweig unter der Hinterlegungsnummer DSMZ ACC 2473 hinterlegt worden ist, die stabil mit einem Plasmid nach Anspruch 6 transfiziert oder mit einem Virus nach Anspruch 6 infiziert ist.

8. Verfahren zur Identifizierung von Substanzen, die bei eukaryontischen Zellen eine Differenzierung auslösen können, mit den Schritten:
a) Inkubieren von transfizierten Zellen mit einem DNA-Konstruktes, das ein Fusionsgen unter der Kontrolle eines Promotors enthält, wobei das Fusionsgen mindestens ein Resistenzgen und mindestens ein Reportergen sowie zumindest ein Gen enthält, das für ein Polypeptid kodiert, das für die transfizierte Wirtszelle eine in geringem Masse toxische Wirkung entfaltet, in einem Kulturmedium mit einer dem Resistenzgen entsprechenden Selektionssubstanz;
b) Überimpfen der inkubierten Zellen aus Schritt a) in ein Kulturmedium, dem die Selektionssubstanz fehlt, und Inkubation der übergeimpften Zellen für cirka 5 bis cirka 100, vorzugsweise cirka 24 h;
c) Zugabe einer zu identifizierten Substanz zu dem Kulturmedium der Zellen aus Schritt b) und weiter Inkubation für cirka 1 bis cirka 5, vorzugsweise cirka 2 Tage; und
d) Vergleichen der inkubierten Zellen aus Schritt c) mit Zellen aus Schritt b) auf erhöhte Expression des Reportergens.

## Claims

1. The use of a DNA construct,
which contains a fusion gene under the control of a promoter,
wherein the fusion gene contains at least one resistance gene and at least one reporter gene, and at least one gene which codes for a polypeptide,
which releases a slightly toxic effect for the transfected host cell to identify substances in a test system that trigger hypo- or demethylation or histone acetylation in eukaryotic cells.

2. The use of a DNA construct according to claim 1, **characterized in that** the promoter is selected from CMV promoter, RSV promoter, cellular promoters of tumor suppressor genes and promoters of differentiation genes.

3. The use of a DNA construct according to claim 1 or 2, **characterized in that** the reporter gene is selected from GFP, LacZ, luciferase.

4. The use of a DNA construct according to one of the claims 1 to 3, **characterized in that** the gene that codes for a polypeptide which releases a toxic effect for the transfected host cell is selected from GFP, cytosine deaminase, HSV thymidine kinase.

5. The use of a DNA construct according to one of the claims 1 to 4, **characterized in that** the resistance gene is selected from hygromycin resistance gene, neomycin phosphotransferase gene, puromycin-N-acetyltransferase gene.

6. The use of a DNA construct according to one of the claims 1 to 5, **characterized in that** the DNA construct is contained in a plasmid or virus.

7. The use of a DNA construct according to one of the claims 1 to 5, **characterized in that** the eukaryotic cell is selected from a human cell, in particular cell line U87-HGFP which was deposited in accordance with the Budapest Treaty at the DSMZ in Braunschweig, under deposition number DSMZ ACC 2473 on 09 November 2000, which is stably transfected with a plasmid according to claim 6 or is infected with a virus according to claim 6.

8. A method for identifying substances which can induce differentiation in eukaryotic cells, comprising the steps:
a) Incubate transfected cells in a culture medium having a selection substance corresponding to the resistance gene, said transfected cells comprising a DNA construct which contains a fusion gene under the control of a promoter, wherein the fusion gene contains at least one resistance gene and at least one reporter gene and at least one gene which codes for a polypeptide which releases a slightly toxic effect for the transfected host cells;
b) Innoculate a culture medium lacking the selection substance with the incubated cells from step a), and incubate the innoculated cells for approximately 5 hours to approximately 100 hours, preferably approximately 24 hours;
c) Add a substance to be identified to the culture medium of the cells from step b) and continue to incubate for approximately 1 hour to approximately 5 hours, preferably approximately 2 days; and
d) Compare the incubated cells from step c) with cells from step b) for increased expression of the reporter gene.

## Revendications

1. Utilisation d'un produit de construction d'ADN,
qui contient un gène de fusion sous le contrôle d'un promoteur,
le gène de fusion contenant au moins un gène de résistance et au moins un gène rapporteur ainsi qu'au moins un gène qui code pour un polypeptide,
qui développe une activité toxique à un faible degré pour la cellule hôte transfectée,
pour l'identification, dans un système d'essai, de substances qui déclenchent une hypométhylation ou une déméthylation ou acétylation d'histones chez des cellules eucaryotes.

2. Utilisation d'un produit de construction d'ADN, selon la revendication 1, **caractérisée par le fait que** le promoteur est choisi parmi le promoteur CMV, le promoteur RSV, les promoteurs cellulaires de gènes suppresseurs de tumeurs et les promoteurs de gènes de différenciation.

3. Utilisation d'un produit de construction d'ADN, selon l'une des revendications 1 ou 2, **caractérisée par le fait que** le gène rapporteur est choisi parmi la GFP, LacZ, la luciférase.

4. Utilisation d'un produit de construction d'ADN, selon l'une des revendications 1 à 3, **caractérisée par le fait que** le gène qui code pour un polypeptide qui développe un effet toxique pour la cellule hôte transfectée est choisi parmi la GFP, la cytosine désaminase, la thymidine kinase du HSV.

5. Utilisation d'un produit de construction d'ADN, selon l'une des revendications 1 à 4, **caractérisée par le fait que** le gène de résistance est choisi parmi le gène de résistance à l'hygromycine, le gène de néomycine phosphotransférase, le gène de puromycine-N-acétyle transférase.

6. Utilisation d'un produit de construction d'ADN, selon l'une des revendications 1 à 5, **caractérisée par le fait que** le produit de construction d'ADN est contenu dans un plasmide ou un virus.

7. Utilisation d'un produit de construction d'ADN selon l'une quelconque des revendications 1 à 5, **caractérisé par le fait que** la cellule eucaryote est choisie parmi une cellule humaine, en particulier la lignée cellulaire U87-HGFP, qui a été déposée le 09/11/2000 selon le Traité de Budapest auprès du DSMZ à Braunschweig sous le numéro de dépôt DSMZ ACC 2473, qui est transfectée de façon stable par un plasmide selon la revendication 6 ou est infectée par un virus selon la revendication 6.

8. Procédé d'identification de substances qui peuvent déclencher une différenciation de cellules eucaryotes, comprenant les étapes suivantes :
a) incubation de cellules transfectées par un produit de construction d'ADN, qui contient un gène de fusion sous le contrôle d'un promoteur, le gène de fusion contenant au moins un gène de résistance et au moins un gène rapporteur ainsi qu'au moins un gène qui code pour un polypeptide qui développe un effet toxique à un faible degré pour la cellule hôte transfectée, dans un milieu de culture contenant une substance de sélection correspondant au gène de résistance ;
b) inoculation des cellules incubées provenant de l'étape a) dans un milieu de culture qui ne contient pas la substance de sélection, et incubation des cellules inoculées pendant environ 5 à environ 100, de préférence environ 24 h ;
c) addition d'une substance à identifier au milieu de culture des cellules provenant de l'étape b) et nouvelle incubation pendant encore 1 à environ 5, de préférence environ 2 jours ; et
d) comparaison des cellules incubées provenant de l'étape c) avec des cellules provenant de l'étape b) pour l'expression augmentée du gène rapporteur.
